# EUROPEAN PATENT APPLICATION

(11) **EP 0 949 269 A1**
(43) Date of publication of application: **13.10.1999**
(21) Application number: 98106089.0
(22) Date of filing: 02.04.1998
(51) Int. Cl.: C07K 14/435, C12N 15/62, G01N 21/64, G01N 33/50

(54) **Biosensor protein**

(71) Applicant: SymBiosis GmbH, 69115 Heidelberg (DE)
(72) Inventor: The designation of the inventor has not yet been filed
(74) Representative: Böhm, Brigitte, Dipl.-Chem. Dr.

(57) **Abstract**

A biosensor protein according to the invention comprises a fusion protein of a) a protein of interest containing a hydrophobic region, or a part thereof, and b) at least the chromophore part of the GFP protein, wherein the GFP chromophore part b) is located within the hydrophobic region of the protein a), and protein a) is sensitive to a signal leading to a conformational change or change of the environment of the chromophore part such that the chromophore's optical properties are changed. Further subjects of the invention are recombinant DNA sequences encoding such biosensor proteins, cell lines which are stably transfected with such recombinant DNA sequences, processes for the determination of the presence or absence of an effector molecule in a sample and test kits useful in such processes.

## Description

The present invention is concerned with biosensor proteins comprising a fusion protein of a protein of interest containing a hydrophobic region or a part thereof and at least the chromophore part of the GFP protein, recombinant DNA sequences encoding said biosensor proteins, cell lines that are stably transfected with such a recombinant DNA sequence and constitutively expressing a biosensor protein according to the invention.

The invention is further concerned with processes for determining the presence or absence of an effector molecule in a sample and a biosensor system kit containing biosensor molecules in appropriate vessels to determine the effect of substances on the biosensor proteins as well as the use of the biosensor system kit for the rapid screening of a plurality of samples for the presence or absence of substances with a certain effect or properties.

Green fluorescent proteins (GFPs) are a source of light in several bioluminescent species as a result of energy transfer. These GFP proteins are a unique class of proteins involved in bioluminescence in many unidria. The GFP proteins act as energy transfer acceptors, receiving energy from either a luciferase-oxiluciferin complex or a Ca²⁺-activated photoprotein, depending on the organism. Upon specific stimulation of the organism the green fluorescent proteins emit a green light. It is common for green fluorescent proteins that they contain a covalently attached chromophore that is composed of several amino acid residues within the polypeptide.

One such GFP protein of interest is the one from Aequorea victoria, a Pacific Northwest jellyfish. This protein in purified form has spectral properties similar to those of the protein expressed in vivo, absorbing blue light and emitting green light which is detectable using a fluorescence microscope. The spectral characteristics of GFP are due to a covalently bound chromophore that results from the spontaneous cyclisation and oxidation of the sequence -Ser⁽⁶⁵⁾ or Thr⁽⁶⁵⁾-Tyr⁽⁵⁶⁾-Gly⁽⁶⁷⁾ and requires the native protein folding for both formation and fluorescence emission. The whole wildtype GFP protein is a stable, proteolysis-resistant single chain protein of 238 residues and has two absorption maxima at about 395 and 475 nm. Properties and crystal structure of GFP from Aequorea victoria are described in detail by Ormö et al., Science, Vol.273, 1392-1395 (1996), Cody et al., Biochemistry 32, 1212-1218 (1993) and Misteli and Spector, Nature Biotechnology, Vol.15, 961-963 (1997). These articles also refer to earlier work on GFPs.

The fluorescence of GFP proteins has already been used as a reporter of gene expression and protein localization, Caine et al., Biotechniques 19, 650-655 (1995), as a tool for studying ion channel expression and function, Marshall et al., Neuron, Vol.14, 211-215 (1995) and for studying the cyclic GMP binding domain of the cyclic GMP-gated ion channel, Kumar and Weber, Biochemistry 31, 4643-4649 (1992).

An overview of applications of green fluorescent protein in cell biology and biotechnology is given by Misteli and Spector (supra).

From the above publications it is clear that GFP protein can be a useful tool in cell biology as some kind of reporter protein. However, applications envisaged so far seem to relate only to singular aspects and do not reveal any common scheme for making use of the fluorescent properties of GFP in a diagnostic or development field or quality control. Therefore, it was the object of the invention to provide possibilities of testing substances in view of their influence on other molecules and to provide simple means for making a statement on the result of the test. It was another object of the invention to provide a possibility of studying the effect of changes in structure or other properties of a protein in view of its behaviour towards substances like ligands, substrates etc. compared to non-mutated proteins.

This object was achieved by the development of a biosensor protein comprising a fusion protein of a) a protein of interest containing a hydrophobic region or a part thereof and b) at least the chromophore part of the GFP protein, wherein the GFP chromophore part b) is located within the hydrophobic region of the protein a), and protein a) is sensitive to a signal leading to a conformational change or change of the environment of the chromophore part such that the chromophore's optical properties are changed.

The biosensor protein of the invention contains, on the one hand, a protein which is to be tested for his reaction to a signal, whereby said protein contains a hydrophobic region. On the other hand, the biosensor protein of the invention contains the chromophore part of the GFP protein, which, within the scope of this invention, means that a sufficiently large part of the GFP protein is present so that optical properties which can be detected are conferred. Within the scope of this invention the chromophore part of the GFP protein is integrated in the protein to be tested, i.e. within the hydrophobic region of said protein.

The protein to be examined reacts to signals in such a way that the GFP chromophore is influenced to the effect that it changes its optical properties. The reaction of the protein to the signal may lead either to a conformational change or to another change of the environment of the chromophore part, which again results in a change of the optical properties.

The chromophore of the GFP protein is within the native GFP protein, enwrapped by the remaining parts of the protein in a cylindrical shape, so that the chromophore is captured almost completely in a hydrophobic environment. In said conformation the optical properties of the GFP protein, its luminescence, can be observed, and luminescence remains almost constant. If the structure changes, however, the optical properties change as well. The present invention makes use of this by introducing the GFP protein, or part containing the chromophore thereof, into an environment of the chromophore similar to that provided by the native GFP. Through this, detectable optical properties can be maintained even in the case of such fusion protein, whereby the strength of the optical properties does not necessarily have to be identical with that of the GFP protein. However, an optical signal still must be detectable within the scope of this invention.

In the case of the biosensor proteins of the invention, too, conformational change or other change of the environment of the chromophore part leads to a change of the optical properties. If there is a signal, to which the protein to be tested reacts, with the environment of the chromophore part changing, this leads to a change of the optical properties.

Within the scope of this invention, instead of a complete protein to be tested only part thereof may be present, however, on condition that said part, too, contains at least part of the hydrophobic region of the protein and a change of the optical properties of the chromophore is also effected by the signal through change of the environmental conditions. The change of the environmental conditions may also manifest itself in a conformational change. In a preferred embodiment of the invention the signal is an effector molecule which directly or indirectly causes a change of the environment of the chromophore part of the biosensor protein according to the invention. Thus, the presence of said signal can be detected via the changes of the optical properties and, especially preferred, it is the binding of an effector molecule.

Said effector molecule which is the signal for change or conformational change in the biosensor protein can, for example, be a ligand binding to a receptor protein of interest, a substrate binding or reacting with an enzyme, an antibody binding to an antigen or another substance interacting with the protein. In preferred embodiments of the invention the biosensor protein includes ion channels, enzymes, receptor molecules, MDR (multi-drug-resistance)-molecules, drug resistance molecules, ATPases, steroids or antigens or parts of such proteins as protein a).

The chromophore part of the GFP protein present in the biosensor protein of the invention preferably comprises at least a hexapeptide from the chromophore region of the GFP protein. From Cody et al., supra, it is already known that, in particular, a hexapeptide confers the optical properties of the GFP protein from Aqueorea victoria. In an especially preferred embodiment, the biosensor protein of the invention contains amino acids 61-68 of GFP. However, the chromophore part may also contain further parts of the GFP protein, as long as this does not impair the sensitivity to a signal by introducing too large a foreign peptide into the hydrophobic region of the protein to be tested.

It is understood that the biosensor protein of the invention quite generally concerns a combination of a protein containing a hydrophobic cavity with an optically active part of the GFP protein and, therefore, biosensor proteins of the invention can be produced using any proteins to be tested, as long as they meet the afore-described requirements. Insofar, the biosensor protein of the invention, on the one hand, serves to study properties such as binding capacity, effects of binding, influence of the presence of non-binding substances that in some other way exert an influence upon the protein as well as for mechanistic studies on the protein to be examined. On the other hand, the biosensor protein of the invention also serves to examine substances and preferably a plurality of substances (for example, via high throughput screening) for their influence on the protein of interest, whereby also changes in the protein structure that are introduced to protein part a) can be examined for resulting changes of behavior towards the substances. In particular, biosensor proteins according to the invention are especially suited for studies, by means of which substances are tested for their environmental harmfulness, toxicity, possible therapeutic use and the like. Another possible application of the biosensor proteins of the invention is detection of the presence or absence of substances in a sample, wherein said substances are supposed to produce a change leading to a determinable effect with regard to the optical properties. In summary, the present invention allows to provide biosensor proteins which are of great interest for a plurality of determinations and tests in the biotechnological, pharmaceutical and ecological field.

Recently, GFP protein mutants have been described which have increased fluorescence and partly also increased stability towards outside influences (e.g. Tsien et al., US-PS 5,625,048). Such mutants and their chromophores, respectively, also can be used within the framework of the present invention, however, on condition that their luminescence is still influenced by conformational changes in the same way or in a similar way as in the case of the wildtype protein. Mutants which do not exhibit any change in luminescence after changing the environment of the chromophore are unsuitable at any rate. In the case of other mutants their suitability depends on the intensity of luminescence and the extent of change in luminescence upon change of the environmental conditions. What is essential for the invention is a measurable difference between the luminescence of the biosensor of the invention with and without the influence of an effector.

It is preferred within the scope of this invention to introduce the biosensor proteins into an artificial environment or associate them with an artifical environment, with artificial cell membranes or polymer molecules being especially preferred. Said association or incorporation facilitates the use of the biosensor proteins, whereby it it conceivable to fix biosensor proteins to polymer membranes, to contact them with substances to be tested and to effect separation by simply taking out the polymer membrane including the biosensor proteins and then detecting the influence of a substance via optical properties. A purposeful arrangement of biosensor proteins in such artificial environments facilitates both the detection and the examination of a plurality of substances at the same time.

Further, incorporation or association with such artificial environment might mimic natural conditions for the proteins of interest, especially, for example, when ion channels are being studied or used. A further object of the invention is a recombinant DNA sequence encoding a biosensor protein according to then invention, wherein it contains a first DNA sequence encoding a protein of interest, containing a hydrophobic region, or a part thereof, and a second DNA sequence encoding a GFP chromophore peptide, and wherein the second DNA sequence is inserted into the first DNA sequence at a location that encodes the hydrophobic region of the protein of interest, and wherein the protein encoded by the first DNA sequence or part thereof, upon receiving a signal, displays a conformational change or change of the environment of the chromophore part such that the chromophore's optical properties are changed. The recombinant DNA sequence of the invention codes for the biosensor fusion protein of the invention. The recombinant DNA consists of at least two partial DNA sequences, whereby the second DNA sequence is integrated at a site which codes for the hydrophobic region of the protein encoded by the first DNA sequence. It is also possible within the scope of the invention that the first DNA sequence codes only for a part of the corresponding protein, whereby, however, the requirements described above in the case of the biosensor protein of the invention must be met. The first partial sequence of the recombinant DNA of the invention codes for a protein reacting to a signal by a conformational change or a change of the environment in the hydrophobic region, whereby a GFP chromophore peptide which is encoded by the second DNA sequence is inserted in said hydrophobic region. Within the scope of the invention the respective signal preferably is the presence or the binding of a effector molecule, whereby an effector molecule can be any molecule leading to a corresponding environmental or conformational change. Such effector molecules, like ligands, substrates, antibodies or the like are the signals, which the protein encoded by the recombinant DNA of the invention preferably reacts to. Accordingly, the proteins encoded by the first DNA sequence of the recombinant DNA of the invention preferably are ion channels, enzymes, receptor molecules or parts thereof containing the hydrophobic region at least partly, and besides also showing a response to the signal.

According to the present invention the second DNA sequence codes for a part of the GFP protein which has detectable optical properties. According to the invention this is preferably at least a hexapeptide of the chromophore of GFP, and more preferably the hexapeptide described in Cody et al., supra and, especially preferred, the second DNA sequence of the invention codes for amino acids 61-68 of the GFP protein.

The recombinant DNA sequence of the invention is useful for the expression of the biosensor protein of the invention and, to this end, can be present in any form allowing expression of the biosensor protein. Expression of the recombinant DNA is possible both in such a way that expression takes place in a cell which does not secrete the protein, but the latter remains in the cytoplasm, and then tests are made on whole cells. To this end, respective signal sequences can be added which, for example, also cause association of the proteins formed with membranes. On the other hand, recombinant expression of the DNA sequence is also possible, whereby the proteins formed are either secreted by the cells and can be recovered from the culture medium, or recovered after rupture of the cell membranes or cell walls.

In order to effect secretion respective signal or/and propeptide sequence can be added as well to the recombinant DNA sequence of the invention. Besides, expression of recombinant DNA is possible in the usual manner known to a skilled artisan. The recombinant DNA sequence can be incorporated in any vector systems, by means of which expression takes place. It is a matter of course to a skilled artisan to use respective control and regulatory sequences like, for example, promoters, operators and binding sites for inducers or inhibitors. Suitable vector systems comprise all systems suited for the expression of a recombinant DNA sequence, such as prokaryotic or eukaryotic vectors, plasmids, cosmids, viral expression systems and the like. Furthermore, expression systems are suited which lead to heterologous recombination and, thus, integration of the recombinant DNA in the cellular genome. By means of homologous recombination, of course, also cells can be obtained which either produce the protein and retain it in their cytoplasm as well as cells secreting the protein in the culture medium.

A further subject matter of the present invention, therefore, is a cell line which is stably transfected with a recombinant DNA sequence, as described above. It is especially advantageous thereby if the non-transfected cell line, into which the recombinant DNA is to be introduced, does not express a protein corresponding to the protein to be examined or part thereof (part a) of the biosensor protein).

The cell line of the invention can be both a eukaryotic and a prokaryotic cell line. The cell line of the invention can contain the DNA sequence coding for the biosensor protein of the invention either stably integrated in the cell genome, or contain it as a plasmid. The cell lines of the invention are characterized in that they produce the biosensor protein of the invention either constitutively or after induction or derepression. It is especially preferred within the scope of the invention that the cell line of the invention expresses the biosensor protein constitutively. The cell lines of the invention either secrete the biosensor protein of the invention into the medium, or biosensor protein accumulates in the cytoplasm of the cell lines. In the latter case it is of advantage, if regulator sequences are integrated which avoid that the cell dies by excess accumulation of biosensor protein in the cells. Pertinent regulator sequences which also can be introduced into the cell by means of recombinant DNA technology are known to a skilled artisan and described, for example, by Sambrook et al., Molecular Cloning (1989), Cold Spring Harbor Press, N.Y.

A further subject matter of the invention is a method for determining the presence or absence of an effector molecule in a sample, whereby the sample is contacted with a biosensor protein of the invention and thereafter the optical properties of the biosensor protein are screened for changes. The optical properties, in particular, are luminescence of the GFP protein which can be determined easily by using a fluorescence emission spectrometer.

The sample to be examined can be used both in liquid and solid or gaseous form as long as sufficient contacting of the biosensor protein is ensured. It is especially preferred to operate with liquid samples.

Another object of the invention is a method for determining the influence exerted by a substance on a biosensor protein according to the invention, wherein again a sample containing said substance is contacted with the biosensor protein, and resulting changes of optical properties are monitored. As for the other method of the invention, the sample can be used in liquid, solid or gaseous form as long as sufficient contact is ensured for the substance to be able to influence the biosensor protein.

The methods of the invention allow to test samples containing specific substances in a simple manner for their influence on the protein of interest. It is also possible thereby to examine samples, in the case of which it is not known whether and/or which substances they contain. As soon as they exert an influence on specific proteins, which can be determined by means of the change of the optical performance of the biosensor proteins of the invention, respective samples can be further examined and the actual effector molecules can be detected. Vice versa, it is conceivable to examine a plurality of substances as to whether they have an influence on particular proteins, whereby, for example, election of suitable substances for drug development and other systematic studies are conceivable.

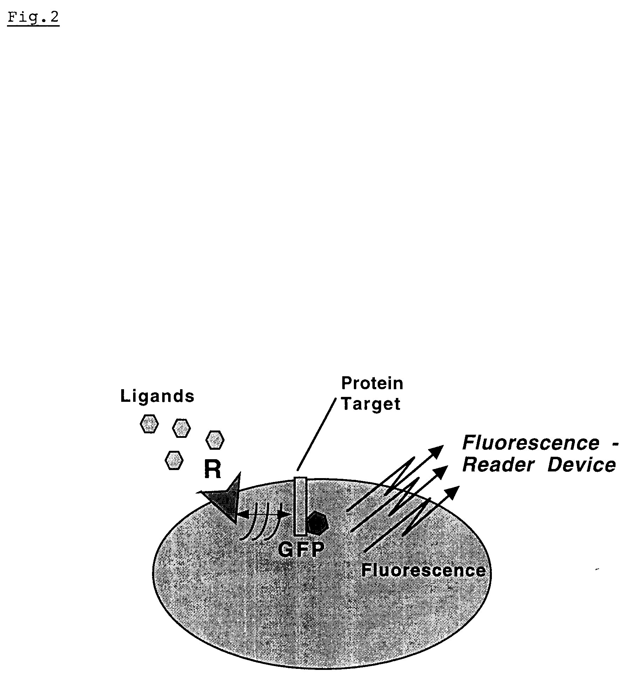

The influence exerted by the effector molecule need not necessarily influence the protein part a) of the biosensor protein of the invention directly. It is also possible to use an indirect assay situation, wherein the effector molecule leads to an intracellular signal (second messenger) that in turn is detected by the biosensor protein of the invention. An example of such second messenger detection is given in Example 2, Fig.2.

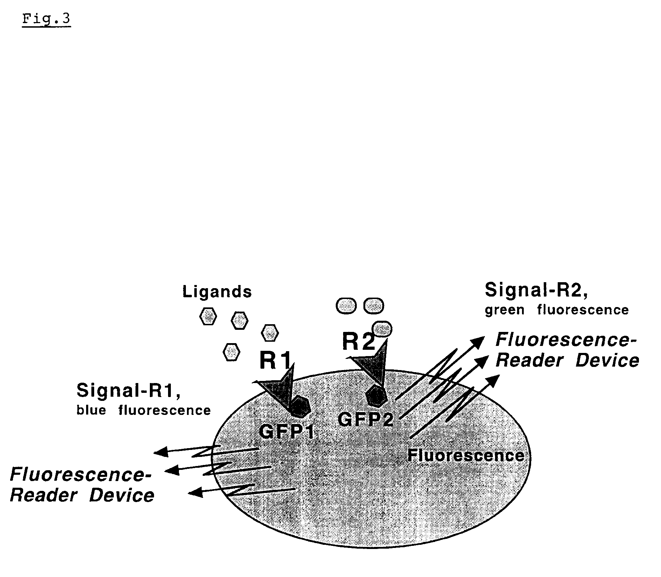

It is also possible to compare the influence of the same effector molecule or different effector molecules on different biosensor proteins that are preferably contained in the same cell line. The luminescence of such different biosensor proteins then can be compared and conclusions can be drawn about interactions. An example of such embodiment is shown in Example 3, Fig.3.

The methods of the invention allow to examine a plurality of substances in a rapid and simple manner, whereby simultaneous performance of a plurality of tests is made possible, for example, by modern pipetting apparatuses and high throughput screening techniques. The methods are especially preferred and suitable for determining effector molecule properties involving binding of the effector molecule to the biosensor proteins of the invention. The methods according to the invention can be used beneficially in many technological fields, as for example diagnostics or environmental technology.

The biosensor proteins used in the process of the invention preferably are obtained by expression of a recombinant DNA of the invention. Isolated biosensor proteins can be used, whereby it is again also of advantage to use proteins associated with or incorporated in an artificial environment. Said artificial environment preferably is an artificial cell membane or a polymer.

In a further preferred embodiment, however, the process of the invention also can be performed with cell lines of the invention that contain the expressed biosensor proteins in their cytoplasm, whereby the samples to be examined can be added directly to the cell lines, and thereafter the optical properties of the cell as a whole are determined.

A further subject matter of the invention is a biosensor system kit containing in suitable vessels a plurality of biosensor proteins of the invention in an environment suitable for preserving their optical properties. The biosensor system kit of the present invention can contain either the same or different biosensor proteins, whereby a plurality of samples or substances can be examined with the same biosensor proteins and the influence of a substance on a plurality of different proteins can be studied with sensor proteins which are different in view of their part a) protein.

The biosensor system kit of the invention, furthermore, can contain the biosensor proteins either in expressed form already, or, preferably, the biosensor system kit contains an expression system containing a recombinant DNA of the invention for expressing the biosensor proteins of the invention as well as means required for effecting expression. In view of this preferred embodiment it is possible, for example, to provide frozen or otherwise preserved cells which, after reactivation, express the biosensor proteins of the invention. The biosensor system kits of the invention can contain intact cells constitutively expressing the biosensor proteins of the invention or cells only expressing after induction or expressing as long as no inhibitor is added. Respective inducers or inhibitors are preferably also included in the biosensor system kits in such cases. Further, the biosensor system kits preferably include the solutions required for reactivating preserved expression systems. The biosensor system kits preferably also contain necessary buffer substances or culture media, as far as this is required due to the expression system used.

The biosensor system kits of the invention are useful for the rapid screening of a plurality of samples for the presence or absence of substances with certain effector properties or the determination of the influence of substances on the part a) protein of biosensor proteins of the invention. Use according to the invention, therefore, is in accordance with the methods of the invention already discussed in detail above, whereby the use of the biosensor system kits of the invention enables a user to carry out determinations in a simple and rapid manner, with the biosensor system kit of the invention preferably providing him with all components required.

In summary, providing the biosensor proteins of the invention, recombinant DNA sequences coding therefor, cell lines expressing the biosensor proteins, the methods of the invention as well as the biosensor system kits of the invention for the first time offers a systematic way of studying substances for their influence on particular proteins or/and testing samples for the presence of particular substances in a simple and rapid manner. Any further or preferred applications are revealed to a skilled artisan by the present description.

The following general Examples, in combination with the drawings, are intended to further illustrate the invention.

### Example 1

### Detection of Ligand Binding

An (optionally modified) GFP containing at least the chromophore is fused into a hydrophobic region of a membrane receptor protein so that the ligand binding induces changes in fluorescence. Binding of specific ligands evokes conformational changes on the receptor sites which lead to changes in light emission that can be detected. The receptor-GFP fusion protein acts as an optical sensor of receptor-ligand interaction.

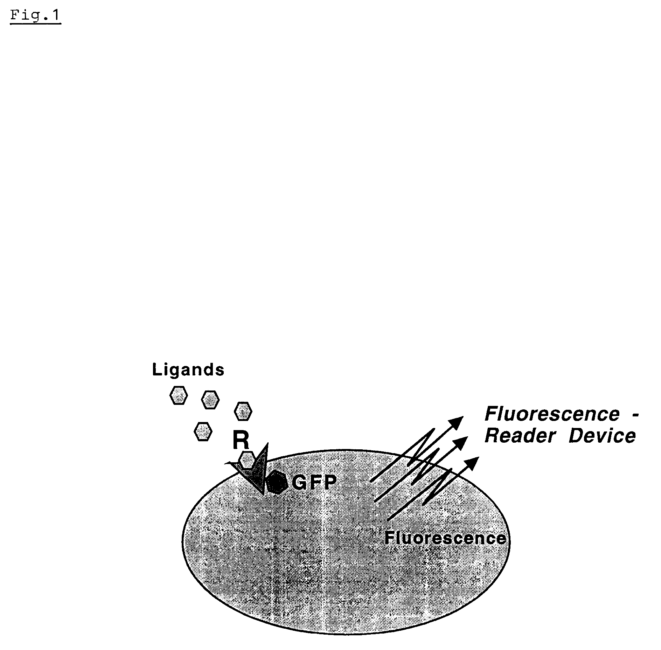

Fig. 1 shows a schematic representation of the test system.

### Example 2

### Detection of Intracellular Effectors

An (optionally modified) GFP containing at least the chromophore is fused to responsive sites in a defined cell type by using a proprietary strategy. This allows to use the cell as a universal tool for the functional detection of e.g. cAMP and cGMP dependent signalling effects of receptor targets. In contrast to reporter gene assays it directly detects the intracellular second messenger concentration, leading to functional responses and a decrease in falsely positive rates.

Fig.2 shows a schematic representation of the mechanism of this embodiment of the invention.

### Example 3

### Definition of State of Effector Protein

A modified GFP type-1 is fused to a receptor subtype-1 and another modified GFP type-2 is fused to a receptor subtype-2. Receptors can be membrane bound or soluble. Such approaches can be used to test the modulation of receptor-1 versus receptor-2. The activation of receptor-1 leads to a fluorescence signal that is different to that emitted by receptor-2. The binding of specific ligands for each of the receptor evokes specific light emission that can be separated from each other.

Fig.3 shows a schematic representation of this embodiment.

## Claims

1. Biosensor protein comprising a fusion protein of
a) a protein of interest containing a hydrophobic region, or a part thereof, and
b) at least the chromophore part of the GFP protein, wherein the GFP chromophore part b) is located within the hydrophobic region of the protein a), and protein a) is sensitive to a signal leading to a conformational change or change of the environment of the chromophore part such that the chromophore's optical properties are changed.

2. Biosensor protein according to claim 1, wherein the signal is presence and/or binding of an effector molecule.

3. Biosensor protein according to claim 2, wherein the effector molecule is a drug, a ligand, a substrate, an antibody or an antigen.

4. Biosensor protein according to any of the foregoing claims, wherein protein a) is an ion channel, an enzyme, a receptor molecule, an ATPase, an MDR-molecule or a steroid, or a part of such protein, still containing at least part of the hydrophobic region.

5. Biosensor protein according to any of the foregoing claims, wherein part b) comprises at least a hexapeptide.

6. Biosensor protein according to claim 5, wherein it contains amino acids 61 to 68 of GFP.

7. Biosensor protein according to any of the foregoing claims, wherein it is associated with or incorporated into an artificial environment.

8. Biosensor protein according to claim 7, wherein the artificial environment is an artificial cell membrane or a polymer.

9. Recombinant DNA sequence encoding a biosensor protein according to any of claims 1 to 8, wherein it contains
1) a first DNA sequence encoding a protein of interest, containing a hydrophobic region, or a part thereof, and
2) a second DNA sequence encoding a GFP chromophore peptide,
and wherein the second DNA sequence is inserted into the first DNA sequence at a location that encodes the hydrophobic region of the protein of interest, and wherein the protein encoded by the first DNA sequence or the part thereof, upon receiving a signal, displays a conformational change or change of the environment of the chromophore part such that the chromophore's optical properties are changed.

10. Recombinant DNA sequence according to claim 9, wherein the signal is an effector molecule that is present and/or binding to the biosensor protein.

11. Recombinant DNA sequence according to claim 10, wherein the effector molecule is a drug, a ligand, a substrate, an antibody or an antigen.

12. Recombinant DNA sequence according to claims 10 or 11, wherein the first sequence encodes an ion channel, an enzyme a receptor molecule, an ATPase, an MDR-molecule or a steroid, or a part of such protein, still containing at least part of the hydrophobic region.

13. Recombinant DNA sequence according to any of claims 9 to 12, wherein the second DNA sequence encodes at least a hexapeptide chromophore of GFP, preferably aminoacids 61 to 68.

14. Recombinant DNA sequence according to any of claims 9 to 13, wherein it is contained in vector molecule.

15. Recombinant DNA sequence according to claim 14, wherein the vector is a procaryotic or eucaryotic vector, plasmid, cosmid or viral expression system.

16. Recombinant DNA sequence according to any of claims 9 to 15, wherein it further contains regulatory sequences like promoters, operators or binding sites for inducers or inhibitors.

17. Cell line characterized in that it is stably transfected with a recombinant DNA sequence according to any of claims 9 to 16.

18. Cell line according to claim 17, wherein the original cell line does not contain a protein encoded by the first DNA sequence.

19. Cell line according to claim 17 or 18, characterized that it constitutively expresses a biosensor protein according to any of claims 1 to 8.

20. Process for determining the presence or absence of an effector molecule in a sample, comprising contacting the sample with a biosensor protein of any of claims 1 to 8 and measuring whether changes in optical properties occur.

21. Process for the determination of an influence exerted by a substance on a protein of interest, wherein a biosensor protein according to claims 1 to 8 and containing at least a part of the protein of interest as part a) is contacted with said substance and possibly occurring changes in the optical properties of the biosensor protein are monitored.

22. Process according to claim 20 or 21, wherein a plurality of substances is screened for the presence of certain effector properties.

23. Process according to claim 20, 21 or 22, wherein the properties are connected with binding to the biosensor protein.

24. Process according to anyone of claims 20 to 23, wherein the biosensor protein is obtained by expression of a recombinant DNA according to claims 9 to 16.

25. Process according to anyone of claims 20 to 24, wherein a cell line according to claims 17, 18 or 19 is contacted with the sample or the substance.

26. Biosensor system kit, containing in appropriate vessels a plurality of biosensor proteins according to claims 1 to 8 in appropriate environment to preserve their optical properties.

27. Biosensor system kit, containing in appropriate vessels a plurality of expression systems, containing a recombinant DNA according to claims 9 to 16 and means for effecting expression of the biosensor molecules.

28. Biosensor system kit, containing in appropriate vessels a plurality of cell lines according to claims 17, 18 or 19 and, if desired, necessary solutions or media for favouring expression of biosensor molecules.

29. Biosensor system kit according to any of claims 27 or 28, wherein the DNA or cells are provided in preserved form, preferably frozen.

30. Use of a biosensor system kit according to any of claims 26 to 28 for the rapid screening of a plurality of samples for the presence or absence of substances with certain effector properties, or the determination of the influence of substances on the part a) protein of biosensor proteins according to claims 1 to 8.
